# EUROPEAN PATENT APPLICATION

(11) **EP 4 710 993 A1**
(43) Date of publication of application: **18.03.2026**
(21) Application number: 25202025.0
(22) Date of filing: 12.09.2025
(51) Int. Cl.: A61N 1/05, A61B 17/34

(54) **PACING LEAD TOOL**

(30) Priority: 13.09.2024 US 202463694664 P; 11.09.2025 US 202519326591
(71) Applicant: Pacesetter, Inc., Sylmar, CA 91342 (US)
(72) Inventor: Jaconetta, Matt, Sylmar, CA 91342 (US); Zhang, Kai, Sylmar, CA 91342 (US); Chantasirivisal, Steve, Sylmar, CA 91342 (US); Sprowls, Mark, Sylmar, CA 91342 (US); Li, Wenwen, Sylmar, CA 91342 (US); Bornzin, Alexander, Sylmar, CA 91342 (US)
(74) Representative: Barker Brettell Sweden AB

(57) **Abstract**

Embodiments are disclosed of a pacing lead tool (100, 200). The pacing lead tool (100, 200) has a tool body (102) with a proximal body end (114), a distal body end (112), and a tool lumen (110) extending from the proximal body end (114) to the distal body end (112). The tool lumen (110) is adapted to receive a proximal lead end of a pacing lead (152), and is also adapted to receive a stylet (156) therein. A retainer (202, 300, 310, 400, 450, 500, 600, 700, 800, 900) is coupled to, or formed in, the tool body (110) and adapted to engage the stylet (156) when the stylet (156) is inserted in the tool lumen (110). The retainer (202, 300, 310, 400, 450, 500, 600, 700, 800, 900) substantially prevents proximal movement of the stylet (156) until a proximal force applied to the stylet (156) exceeds a threshold force.

## Description

### TECHNICAL FIELD

The disclosed embodiments relate generally to pacing leads for conducting electrical signals to and from target tissue. Specifically, they relate to a pacing lead tool. More particularly, but not exclusively, they relate to a pacing lead tool used to implant pacing leads.

### BACKGROUND

Cardiac pacing by an artificial pacemaker, known more generally as a "biostimulator," provides electrical stimulation of the heart when the natural pacemaker and/or conduction system of the heart fails to provide synchronized atrial and ventricular contractions at healthy rates and intervals. Such antibradycardial pacing provides relief from symptoms, and even life support, for hundreds of thousands of patients. Cardiac pacing can also provide electrical overdrive stimulation to suppress or convert tachyarrhythmias, again supplying relief from symptoms and preventing or terminating arrhythmias that could lead to sudden cardiac death.

Currently available or conventional pacemakers usually perform cardiac pacing using an electrical pulse generator implanted subcutaneously or sub-muscularly in or near the pectoral region of a patient. The operating parameters of the pulse generator are usually interrogated and modified in one of three ways: by a programming device outside the body, via a loosely-coupled transformer with one inductance inside the body and another outside, or via electromagnetic radiation with one antenna inside the body and another outside. The pulse generator usually connects to the proximal end of one or more implanted leads, the distal ends of which contain one or more electrodes for positioning adjacent to the inside or outside wall of a cardiac chamber. The leads have an electrical conductor for connecting the pulse generator to electrodes in the heart. Such electrode leads typically have lengths of 50-80 centimeters. Pacing leads can engage and/or be fixed to an intracardial implant site by an engaging mechanism such as an electrode anchor. For example, the electrode anchor can screw or expand into the myocardium.

Fixed helix leads have become the standard of care for pacing applications such as cardiac physiologic pacing, in which such leads are delivered through fixed or deflectable catheters to specific targets in the His Bundle or the Left Bundle Branch (LBB). Extendable/retractable cardiac pacing leads can also be used in physiological pacing applications. These leads commonly include a stylet lumen, which allows for a stylet to be placed through the lead for support within the target anatomy. Such leads also include a connector pin, which is controlled relative to a lead body during the implant procedure. In some examples, the connector pin can be rotated to extend or retract a helix or other anchor for active fixation at the target tissue. For example, a tool can be clipped onto the connector pin and a physician can hold the lead body in one hand while rotating the tool with another hand to cause rotation of the connector pin or to deploy the connector pin. Accordingly, the helix can be protected within the lead body during delivery to the target site and, after delivery to the site, can be extended to engage the target tissue. A physician can then use the stylet to push the lead through the target tissue to burrow the lead toward a target pacing site.

### SUMMARY

In an aspect, a pacing lead tool includes a tool body and a retainer. The tool body has a proximal body end, a distal body end, and a tool lumen extending in a longitudinal direction from the proximal body end to the distal body end. The tool lumen is adapted to receive a proximal lead end of a pacing lead and a stylet therein. The retainer is coupled to the tool body and adapted to engage the stylet when the stylet is inserted in the tool lumen to resist movement of the stylet.

In an aspect, a pacing lead tool includes a tool body and a retainer. The tool body has a proximal body end, a distal body end, and a tool lumen extending in a longitudinal direction from the proximal body end to the distal body end. The tool lumen is adapted to receive a proximal lead end of a pacing lead and a stylet therein. The retainer is formed in the tool body and adapted to engage the stylet when the stylet is inserted in the tool lumen to resist movement of the stylet.

In an aspect, a pacing lead system includes a pacing lead, a tool body, a stylet, and a retainer. The pacing lead has a proximal lead end, a distal lead end, and a lead lumen extending from the proximal lead end to the distal lead end. The tool body has a proximal body end, a distal body end, and a tool lumen extending from the proximal body end to the distal body end. The tool lumen is adapted to receive the proximal lead end of the pacing lead. The stylet is positioned in the lead lumen of the pacing lead and the tool lumen of the tool body. The stylet has a distal stylet end that is positioned at or near the distal lead end of the pacing lead and has a proximal stylet end that is positioned outside the proximal lead end of the tool body. The retainer is adapted to engage the stylet to resist movement of the stylet.

In an aspect, a pacing lead system includes a pacing lead having a proximal lead end, a distal lead end, and a lead lumen extending from the proximal lead end to the distal lead end. The pacing lead system also includes a pacing lead tool according to above, wherein the proximal lead end is received in the tool lumen.

### BRIEF DESCRIPTION OF THE DRAWINGS

Non-limiting and non-exhaustive embodiments of the present invention are described with reference to the following figures, wherein like reference numerals refer to like parts throughout the various views unless otherwise specified.
FIG. 1A is a diagram of an embodiment of a system for attaching a pacing lead to target tissue.
FIG. 1B is a perspective view of an embodiment of a pacing lead tool.
FIGS. 2A-2E are views of an embodiment of a pacing lead tool that includes an embodiment of a retainer to secure a stylet.
FIGS. 3A-3D are views of an embodiment of a retainer to secure a stylet in a pacing lead tool.
FIGS. 4A-4B are views of embodiments of a retainer to secure a stylet in a pacing lead tool.
FIGS. 5A-5B are views of an embodiment of a retainer to secure a stylet in pacing lead tool.
FIG. 6 is a view of an embodiment of a retainer to secure a stylet in a pacing lead tool.
FIG. 7 is view of an embodiment of a retainer to secure a stylet in a pacing lead tool.
FIG. 8 is view of an embodiment of a retainer to secure a stylet in a pacing lead tool.
FIG. 9 is view of an embodiment of a retainer to secure a stylet in a pacing lead tool.

### DETAILED DESCRIPTION

Embodiments are described of a pacing lead tool for implanting pacing leads. Specific details are described to provide an understanding of the embodiments, but one skilled in the relevant art will recognize that the invention can be practiced without one or more of the described details or with other methods, components, materials, etc. In some instances, well-known structures, materials, or operations are not shown or described in detail but are nonetheless assumed.

Reference throughout this specification to "one embodiment" or "an embodiment" means that a described feature, structure, or characteristic can be included in at least one described embodiment, so that appearances of "in one embodiment" or "in an embodiment" do not necessarily all refer to the same embodiment. Furthermore, the described features, structures, or characteristics can be combined in any suitable manner in one or more embodiments.

As used in this application, directional terms such as "left," "right," "front," "rear," "upper," lower," "top," "bottom," "side," "lateral," "longitudinal," etc., refer to the orientations of embodiments as they are presented in the drawings, but any directional term should not be interpreted to imply or require a particular orientation of the described embodiments when in actual use. The use of relative terms throughout the description can denote a relative position or direction. For example, "distal" can indicate a first direction along a longitudinal axis of a pacing lead tool and "proximal" can indicate a second direction opposite to the first direction. Such terms are provided to establish relative frames of reference, but are not intended to limit the use or orientation of the disclosed devices to a specific configuration described in the various embodiments below.

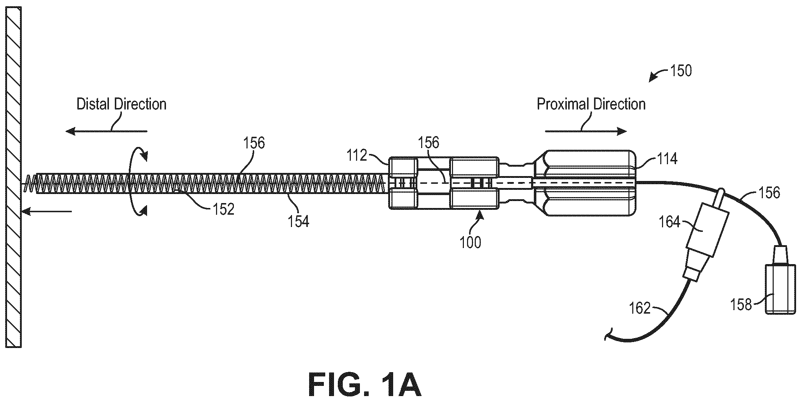

FIG. 1A illustrates an embodiment of a pacing lead system 150 for implanting a pacing lead. The pacing lead system 150 includes a pacing lead tool 100 removably attached to an end of a pacing lead 152 so that the pacing lead emerges from the distal body end 112 of the tool. Pacing lead 152 is in turn positioned within a catheter 154 and the catheter, like the pacing lead, is also removably attached to tool 100. In one embodiment, the pacing lead can be a defibrillation lead used to pace, but in other embodiments it can have different or additional functions.

Pacing lead 152 can include a helical pacing lead. Accordingly, the pacing lead 152 can include a helical fixation element to anchor the lead into a target anatomy. The pacing lead 152 may include a lead lumen into which a stylet 156 can be inserted. Stylet 156 is inserted into tool 100 and pacing lead 152 so that the stylet extends all the way through tool 100 and through the lead lumen of the pacing lead. When positioned correctly, the distal stylet end of the stylet is at or near the distal lead end of the pacing lead. In one embodiment, stylet 156 is a flexible thin wire with enough stiffness that it can be used, by pushing distally from its proximal stylet end, to exert a force, F, on the distal lead end of the pacing lead. A proximal portion of stylet 156 emerges from proximal body end 114 of a tool body of the tool, and a cap 158 is attached at the proximal stylet end of the stylet.

In operation of system 150, the system is first prepared for use. Pacing lead 152 and catheter 154 are removably attached to tool 100 so that both emerge from distal body end 112 of the tool. In some embodiments, stylet 156 can come pre-loaded in the lead lumen of the pacing lead, so that the stylet, pacing lead, and catheter are attached to the tool substantially simultaneously. But if stylet 156 does not come pre-loaded in the lead lumen of the pacing lead, the stylet can be inserted in the tool and fed into the lead lumen of the pacing lead, for instance by pushing the stylet distally from the proximal body end 114 of the tool until the position of the distal stylet end of the stylet is at or near the position of the distal lead end of the pacing lead. In some embodiments stylet 156 is electrically conductive and can be used to provide pacing signals or to measure electrical quantities. In these embodiments, a pacing system analyzer (PSA) wire 162 can be attached to stylet 156 by an alligator clip 164.

Once prepared for use, the distal ends of pacing lead 152, catheter 154, and stylet 156 are inserted into the body of a patient and navigated to tissue 160, where the pacing lead will be implanted. In one embodiment tissue 160 is heart tissue, but in other embodiments it can be another type of tissue. By grasping and turning tool 100, the surgeon applies a torque to pacing lead 152 and catheter 154, causing the pacing lead and catheter to rotate as indicated by the circular arrow in the figure. At the same time, by pushing distally on the portion of stylet 156 that emerges from proximal body end 114, a force, F, is applied by the stylet to the distal lead end of the pacing lead. This combination of rotating the pacing lead and applying a distal force, F, causes the distal lead end of the pacing lead to burrow into tissue 160, thus implanting the pacing lead in the tissue.

When pacing lead 152 has been implanted in the tissue, the stylet is withdrawn from the lead lumen of the pacing lead and tool 100 by pulling it proximally, and catheter 154 is also withdrawn from the pacing lead, leaving the pacing lead behind in the patient. One problem that can occur is that, during an operation, the weight of cap 158, PSA cable 162, and alligator clip 164 can cause stylet 156 to be pulled proximally out of the lead lumen of the pacing lead. When that happens, the distal stylet end of the stylet is no longer at or near the distal lead end of the pacing lead, so that the stylet is less effective or no longer effective in providing the distal force, F, needed to implant the pacing lead in tissue 160.

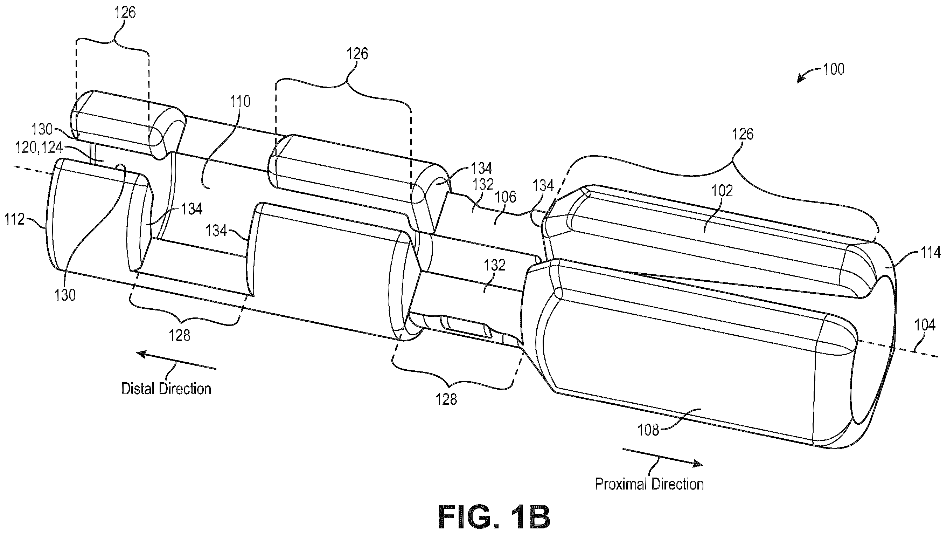

FIG. 1B illustrates an embodiment of a pacing lead tool 100. The pacing lead tool can be mounted on an extendable cardiac pacing lead to convert the lead into a fixed helix cardiac pacing lead during implantation. More particularly, pacing lead tool 100 can constrain a connector pin of the pacing lead relative to a lead body of the pacing lead. The constraint might be torque-limited, however. Application of a torque above a predetermined limit to the pacing lead tool 100, when the pacing lead tool is mounted on the connector pin and the lead body, can cause the pacing lead tool and the connector pin to rotate together relative to the lead body, effectively converting a lead that was an extendable helix into a fixed-helix lead.

In an embodiment, pacing lead tool 100 includes a tool body 102, which can be monolithic or formed from several components connected to each other. The tool body can include a longitudinal axis 104 extending centrally through the body. A body wall 106 of the tool body 102 can have an outer surface that can be gripped by a physician. For example, body wall 106 can define a handle 108 that is disposed about longitudinal axis 104. Handle 108 can have a gripping surface, e.g., a hexagonal, circular, square, elliptical, contoured, or other cross-section, ridges, knurling, etc. to facilitate gripping and twisting of the pacing lead tool 100 by the physician. The handle can be symmetrically disposed about the longitudinal axis 104 or can have an asymmetric distribution about the longitudinal axis. For example, the handle 108 can have a lever extending laterally in a single direction.

In an embodiment, body wall 106 extends around a tool lumen 110 of the pacing lead tool 100. Tool lumen 110 can extend along the longitudinal axis 104 through the tool body 102 from a distal tool end 112 to a proximal tool end 114. The distal tool end 112 can be a distalmost, distally-facing surface of the tool body 102. The proximal tool end 114 can be a proximal-most, proximally-facing surface of the handle 108.

As described below, tool lumen 110 can have segments and subsegments adapted to different purposes. For example, a portion of the tool lumen 110 can be adapted to receive a proximal lead end of a cardiac pacing lead. The cardiac pacing lead can be an extendable pacing lead, and the tool lumen 110 can be sized such that the tool body 102 grips a connector pin of the pacing lead. Furthermore, a portion of tool lumen 110 can be adapted to allow stylets to be exchanged into the pacing lead. The size and shape of the tool lumen can facilitate the functions described below.

Pacing lead tool 100 can include a side slot 120 to expose the tool lumen 110 to a surrounding environment 122. More particularly, side slot 120 can extend through the body wall 106 of the tool body 102 from the tool lumen 110 to the surrounding environment 122. Accordingly, side slot 120 provides an opening between the tool lumen 110 and the surrounding environment 122. Objects such as a stylet or an electrical connector can therefore pass through the side slot 120 into the tool lumen 110.

In an embodiment, side slot 120 can be an intermittent slot 124. More particularly, side slot 120 can include several slot segments 126 longitudinally separated by several intervening connector openings 128. Slot segments 126 and connector openings 128 can combine to form a continuous opening from the proximal tool end 114 to the distal tool end 112. But the opening, although continuous, need not be consistent. For example, a circumferential width of the segments forming the continuous opening can vary over a length of the pacing lead tool 100.

The circumferential width of slot segments 126 of the side slot 120 can be less than the width of the connector openings 128. For example, the circumferential width of the slot segments 126, measured laterally between circumferentially-facing slot segment surfaces 130 of the slot segments 126, can be less than a diameter of the tool lumen 110 exposed through the slot segment 126. The tool lumen 110 exposed through the slot segments 126 can have a diameter of, e.g., 0.135 inch, and the circumferential width of the slot segments 126 can be less than 0.135 inch, e.g., 0.040 inch. By contrast, the circumferential width of connector openings 128, measured laterally between circumferentially-facing connect slot surfaces 132 of the connector openings, can be larger than the circumferential width of slot segments 126. In an embodiment, the circumferential width of connector openings 128 is at least as large as the diameter of tool lumen 110. More particularly, connector openings 128 can extend through body wall 106 to longitudinal axis 104, and thus an entirety of tool lumen 110 can be exposed through connector opening 128.

Given that the connector opening 128 can have a larger circumferential width than the slot segments 126, the connector openings 128 can interrupt the slot segments 126. The interrupted slot segments 126 can have slot segment faces 134 that face longitudinally across the connector openings 128. For example, slot segment faces 134 can face each other through the connector openings 128. The connector openings 128 can therefore be defined between the slot segment faces 134 and the connector opening surfaces 132, and can provide an opening large enough to receive an electrical connector. Furthermore, connector openings 128 can interrupt slot segments 126, which are not large enough to receive the electrical connector, but which can receive a stylet laterally through body wall 106. The continuous opening between distal tool end 112 and proximal tool end 114 accordingly has a varied width that exposes different degrees of the tool lumen over the tool length.

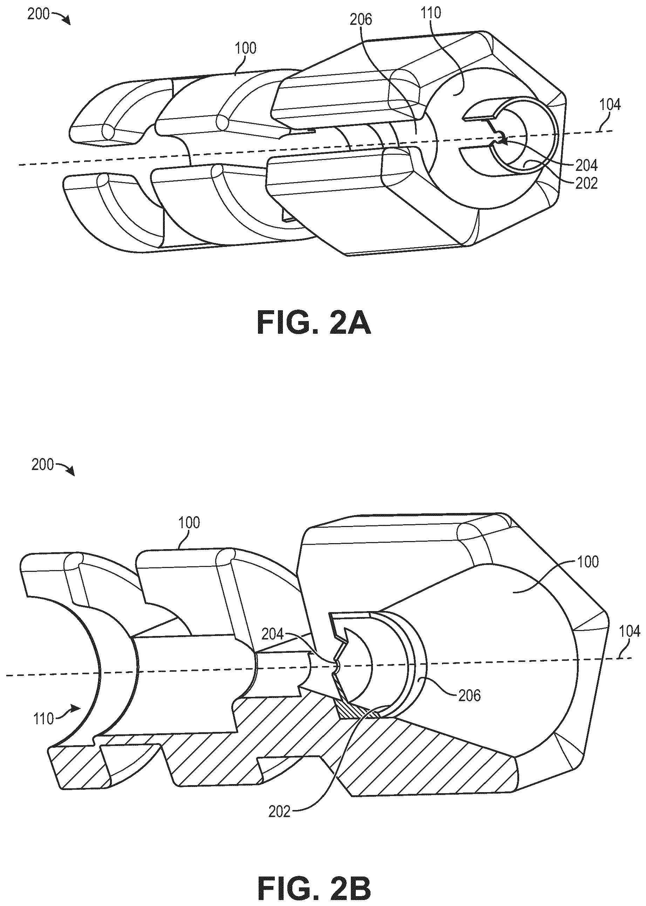

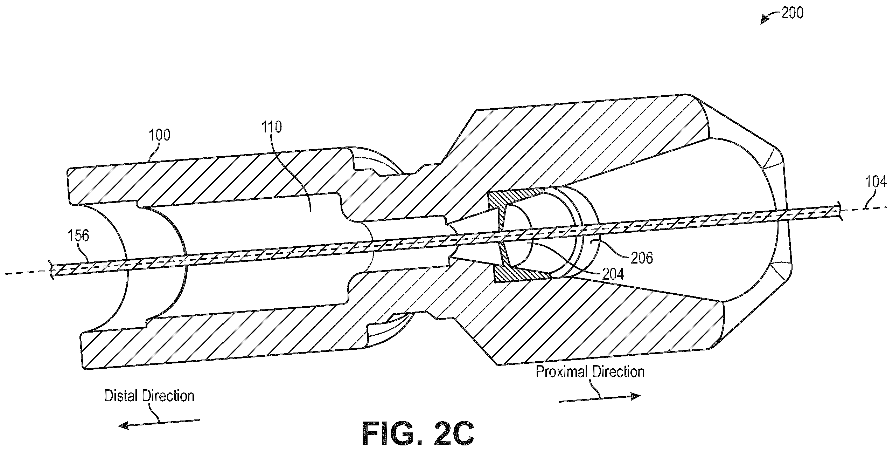

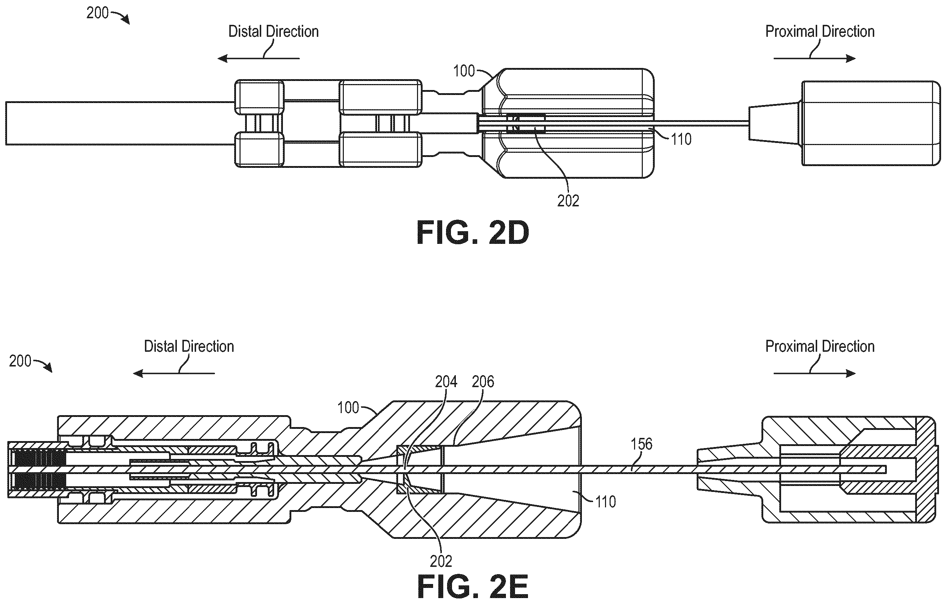

FIGS. 2A-2E together illustrate a pacing lead tool 200 for implanting a cardiac pacing lead. FIGS. 2A-2C are perspective views of tool 200 and FIGS. 2D-2E are top and cross-sectional views, respectively, of tool 200.

Pacing lead tool 200 includes a basic pacing lead tool with an interior lumen. In the illustrated embodiment, the basic tool can be substantially the same as tool 100, illustrated in FIG. 1B and described above, but in other embodiments the basic tool can be configured differently than shown. Tool 100 includes a tool body 102 with a tool lumen 110, and a longitudinal axis 104 extends centrally through both the body and the tool lumen.

Tool 200 includes a retainer to help minimize or eliminate proximal movement of the stylet in response to a longitudinal force (e.g., a proximal force in a proximal direction) applied to the proximal stylet end of the stylet, such as the weight of stylet end cap 158 and PSA cable 162 (see FIG. 1A). In tool 200, the retainer is an insert 202 positioned inside tool lumen 110. Insert 202 is positioned along longitudinal axis 104 and includes an opening 204 to receive a stylet and help retain the stylet in the tool. In the illustrated embodiment, opening 204 is substantially positioned along and aligned with longitudinal axis 104, but in other embodiments the opening can be laterally offset from the longitudinal axis-i.e., offset in a direction normal to the longitudinal axis.

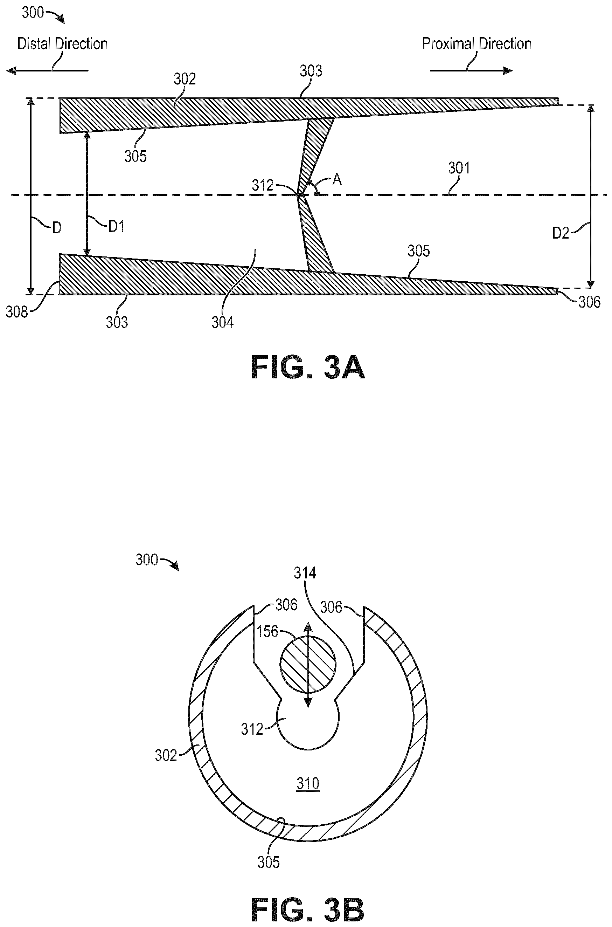

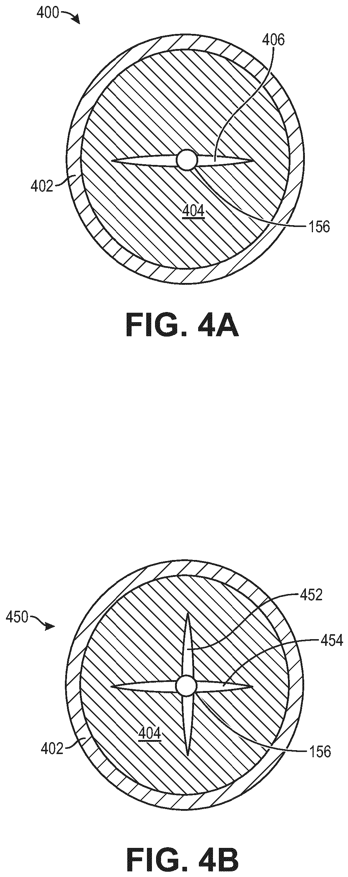

In one embodiment, a notch 206 or other structure can be formed in tool lumen 110 to accommodate and position the insert, so that the insert can be inserted into the notch and secured in place. In an embodiment using a notch, all or part of the notch can be sized and shaped to substantially conform to an exterior surface of the insert, but in other embodiments the notch need not match the size of the insert. Other embodiments need not use a notch inside the lumen, but can instead accommodate the insert in other ways. In one embodiment the insert can be held in notch 206 using an adhesive, but in other embodiments it can be held in the notch in other ways, such as by sizing the notch and the insert to create an interference fit. When a stylet is inserted in tool 200, insert 202 helps, among other things, to secure the stylet in the tool. For example, the insert 202 can engage the stylet when the stylet is inserted in a lumen of the tool to resist movement of the stylet. Embodiments of insert 202 are discussed below in connection with FIGS. 3A-3D and 4A-4B.

FIGS. 3A-3D together illustrate an embodiment of an insert 300 for a pacing lead tool. FIG. 3A is a longitudinal cross-sectional view of insert 300, and FIG. 3B is a cross-section of insert taken substantially along section line B-B in FIG. 3A.

Insert 300 includes a substantially cannular body 302 of outer diameter D. The sidewalls of cannular body 302 are defined by an outer surface 303, an inner surface 305, a proximal insert end 306, and a distal insert end 308. In an embodiment, insert 300 can be made of a flexible material such as silicone, but in other embodiments it can be made of a different flexible material, such as rubber, and in still other embodiments it can be made of a material not generally considered flexible.

An interior insert lumen 304 is defined at least in part by inner surface 305, and the insert lumen extends through the length of the insert from proximal insert end 306 to distal insert end 308. In the illustrated embodiment, insert lumen 304 tapers so that its diameter d1 at the distal insert end is smaller than its diameter d2 at the proximal insert end (i.e., d1 < d2). But in other embodiments insert lumen 304 can taper the opposite way, with diameter d1 larger than diameter d2 (i.e., d1 > d2). Other embodiments with a tapered insert lumen 304 need not taper linearly between the proximal and distal insert ends. And in still other embodiments, insert lumen 304 need not taper at all (i.e., d1 = d2).

A membrane 310 extends inwardly from inner wall 305 toward a central axis 301 of the insert. A hole 312, formed in the membrane, is positioned substantially along, and aligned with, central axis 301. Membrane 310 is oriented such that it forms an angle A relative to central axis 301. In the illustrated embodiment, angle A is generally chosen so that membrane 310 has a distal slant-i.e., so that the center of membrane 310 is at a more distal location than its perimeter. In the illustrated embodiment, then, the result of the distal slant of the membrane is that hole 312 is more distally located than where the membrane joins inner wall 305. In the illustrated embodiment, A is an acute angle (A < 90°). In other embodiments membrane 310 can be normal relative to axis 310 (A = 90°) so that the membrane has no slant. In still other embodiments, membrane 310 can be obtuse relative to axis 301 (A > 90°) and thus have a proximal slant.

FIG. 3B illustrates a cross-section of insert 300 and membrane 310. In the illustrated embodiment, insert 300 is notched-i.e., both insert body 302 and membrane 310 have a notch 314 cut out of them. As a result of notch 314, insert body 302 is not fully cannular and membrane 310 is not fully circular. Other insert embodiments need not have notch 314; in those embodiments body 302 is fully cannular and membrane 310 is circular (see, e.g., FIGS.4A-4B).

Hole 312 in membrane 310 is sized and shaped to receive stylet 156 (see, e.g., FIG. 3B). When the stylet is positioned in hole 312, the hole engages and interacts with the stylet, meaning that at least some part of the stylet is in physical contact with the circumference of the hole so that the hole can exert forces on the stylet, both longitudinally (i.e., proximally and distally along axis 301) and transversely (i.e., normal to axis 301). With this arrangement, the distal force (i.e., force in the distal direction) applied to the stylet is greater than the proximal force (i.e., force in the proximal direction) applied to the stylet. The slit thus allows the stylet to move distally while reducing or eliminating its proximal movement until a longitudinal (e.g., proximal) force applied to the stylet-in one embodiment, by the weight of the part of the stylet that emerges from proximal body end 114 and, if present, stylet cap 158 and PSA cable 162-exceeds a certain threshold. Thus, stylet 156 remains centered in tool 100 and does not move proximally until a force is applied that exceeds the threshold. The force threshold can be exceeded, for instance, by a surgeon pulling the stylet proximally out of the tool. Accordingly, the membrane 310 is a retainer adapted to engage the stylet when the stylet is inserted in the tool lumen to resist movement of the stylet.

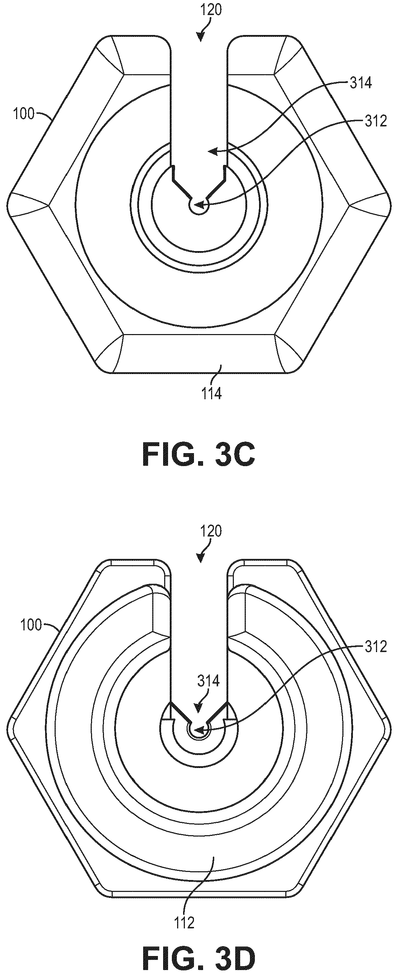

FIGS. 3C-3D illustrate insert 300 when mounted in lead conversion tool 100. FIG. 3C is a view from the proximal insert end, FIG. 3D a view from the distal insert end. Insert 300 is compatible with pacing lead tool 100 (see FIG. 1B), meaning that notch 314 aligns with side slot 120, so that stylet 156 can be inserted in hole 312 and removed from hole 312, and hence inserted and removed from tool 100, by transverse movement (i.e., normal to axis 310), as shown in FIG. 3B by the double-headed arrow.

FIG. 4A illustrates an embodiment of an insert 400. Insert 400 has the same basic construction as insert 300: it includes a substantially cannular body 402 and a membrane 404, both of which are of similar construction to the body and membrane of insert 300 and subject to the same variations in different embodiments. The primary difference between inserts 300 and 400 is that insert 400 does not include notch 314 or hole 312. Without notch 313, body 302 is cannular and membrane 404 is circular. And instead of using hole 312 to engage and interact with stylet 156, membrane 404 includes a slit 406. In the illustrated embodiment slit 406 is substantially horizontal, but in other embodiment it can have a different orientation than shown. As in insert 300, the distal force (i.e., force in the distal direction) applied to the stylet by slit 406 is greater than the proximal force (i.e., force in the proximal direction) applied to the stylet. The slit thus allows the stylet to move distally while reducing or eliminating proximal movement of the stylet until a proximal force applied to the stylet-in one embodiment, by the weight of the part of the stylet that emerges from proximal body end 114 and, if present, stylet cap 158, and PSA cable 162-exceeds a certain threshold. Thus, stylet 156 remains centered in tool 100 and does not move proximally, or moves very little proximally, until a force is applied that exceeds the threshold. The force threshold can be exceeded, for instance, by a surgeon pulling the stylet proximally out of the tool.

FIG. 4B illustrates an embodiment of an insert 450. Insert 450 has the same basic construction as insert 400: it includes a substantially cannular body 402 and a membrane 404, both of which are of similar construction to the body and membrane of insert 400 and subject to the same variations in different embodiments. The primary difference between inserts 400 and 450 is that insert 450 includes multiple slits-in this embodiment, a pair of slits 452 and 454, although other embodiments can include more than two slits. In the illustrated embodiment slit 452 and 454 are substantially perpendicular to each other, but in other embodiments the need not be perpendicular. Also in this embodiment slits 452 and 454 are positioned substantially vertically and horizontally, but in other embodiment the slits can have a different orientation than shown.

As in insert 400, the distal force (i.e., force in the distal direction) applied by slits 452 and 454 to the stylet is greater than the proximal force (i.e., force in the proximal direction) applied to the stylet. The slits thus allows the stylet to move distally while reducing or eliminating proximal movement of the stylet until a proximal force applied to the stylet -in one embodiment, by the weight of the part of the stylet that emerges from proximal body end 114 and, if present, stylet cap 158, and PSA cable 162-exceeds a certain threshold. Thus, stylet 156 remains centered in tool 100 and does not move proximally until a force is applied that exceeds the threshold. The force threshold can be exceeded, for instance, by a surgeon pulling the stylet proximally out of the tool. Accordingly, the insert 400 is a retainer adapted to engage the stylet when the stylet is inserted in the tool lumen to resist movement of the stylet.

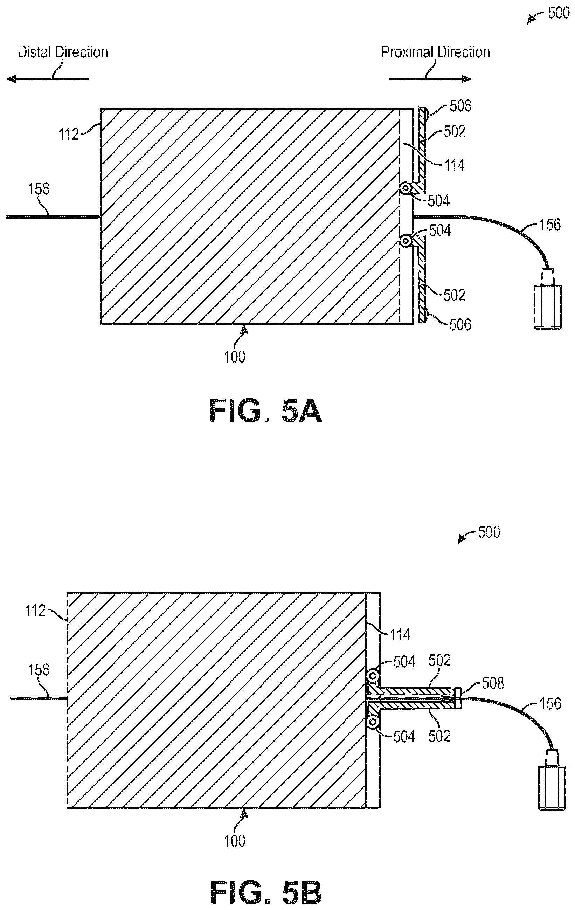

FIGS. 5A-5B together illustrate an embodiment of a retainer 500 for securing a stylet in a pacing lead tool. FIG. 5A shows the retainer in its open position, where the stylet is unsecured, and FIG. 5B shows the retainer in its closed position, where the stylet is secured. Retainer 500 is attached to the proximal body end 114 of a pacing lead tool such as tool 100. Retainer 500 can be attached to proximal body end 114 differently in different embodiments. In one embodiment, retainer number 500 can be attached with adhesives or fasteners. But in another embodiment retainer 500 can be incorporated into tool 100; for instance, in an embodiment where tool 100 is made of molded plastic, retainer 500 can be directly incorporated into the tool by overmolding it into a proximal portion of the tool.

Retainer 500 includes a pair of arms 502, each of which is secured by and rotates about a hinge 504. In the illustrated embodiment each arm also includes a pad 506 to engage the stylet 156, but in other embodiments the pad can be omitted and the arms themselves can engage with the stylet. In embodiments where pads 506 are present, they can be made of a flexible gripping material such as rubber or silicone.

In operation, each arm 502 can rotate about its hinge 504 from an open position shown in FIG. 5A to a closed position shown in FIG. 5B. Hinges 504 are transversely positioned (i.e., positioned normal to the distal/proximal axis) so that when arms 502 are in their closed position they engage stylet 156. In embodiments that include pads 506, when arms 502 are in closed position the pads engage stylet 156. When in closed position, arms 502 can be secured together by a clip 508 or other mechanism.

As with the inserts described above, when in closed position the arms thus allow the stylet to move distally while reducing or eliminating proximal movement of the stylet until a proximal force applied to the stylet - e.g., by the weight of the part of the stylet that emerges from proximal body end 114 and, if present, stylet cap 158, and PSA cable 162-exceeds a certain threshold. Thus, stylet 156 remains centered in tool 100 and does not move proximally until a force is applied that exceeds the threshold. The force threshold can be exceeded, for instance, by a surgeon pulling the stylet proximally out of the tool. Accordingly, the arms 502 provide a retainer adapted to engage the stylet when the stylet is inserted in a lumen of the tool to resist movement of the stylet.

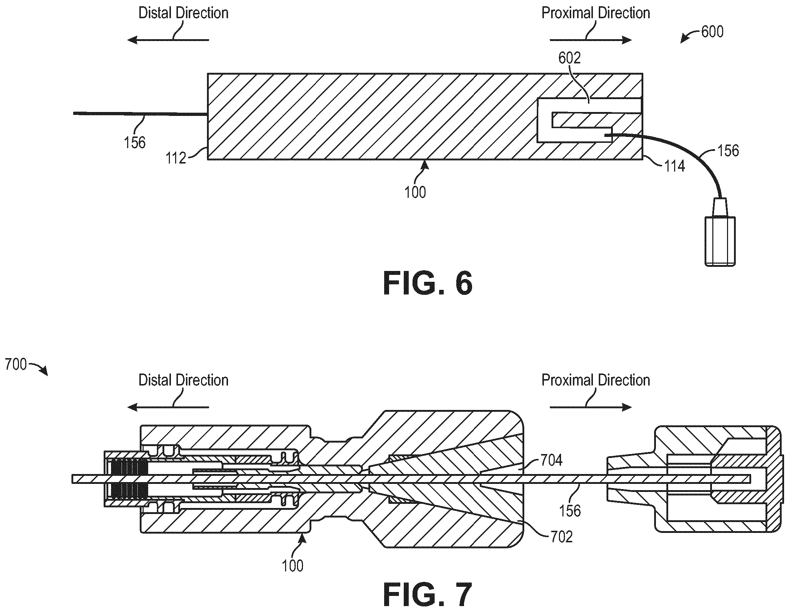

FIG. 6 illustrates an embodiment of a retainer 600 for securing a stylet in a pacing lead tool. In retainer 600, a slot 602 is formed at or near proximal body end 114 of pacing lead tool 100. In the illustrated embodiment slot 602 is J-shaped, but in other embodiments slot 602 can have a different shape than shown. In operation of retainer 600, stylet 156 is simply threaded into slot 602 so that the slot engages the stylet and prevents it from moving in at least the proximal direction. Accordingly, the slot 602 is a retainer, formed in the tool body, and adapted to engage the stylet when the stylet is inserted in the lumen to resist movement of the stylet.

FIG. 7 illustrates an embodiment of a retainer 700 for securing a stylet in a pacing lead tool. In retainer 700, a plug 702 is formed in proximal body end 114 of a pacing lead tool such as tool 100. Plug 702 includes a central opening 704 to receive stylet 156. In one embodiment, plug 602 can be formed by filling part of the lumen with a material, for instance by injecting the material into a proximal portion of the tool and allowing it to at least partially solidify or cure. But in other embodiments the plug can be formed separately and then inserted into the proximal portion. In different embodiments, whether formed by injection of formed separately and then inserted, plug 702 can be made of a gel or one or more flexible materials such as plastic, rubber, or silicone.

In operation of retainer 700, plug 702 functions similarly to the inserts describe above. When stylet 156 is inserted in tool 100 and plug 702, the plug engages and interacts with the stylet. As with the inserts described above, plug 702 allows the stylet to move distally while reducing or eliminating proximal movement of the stylet until a proximal force applied to the stylet-e.g., by the weight of the part of the stylet that emerges from proximal body end 114 and, if present, stylet cap 158, and PSA cable 162-exceeds a certain threshold. Thus, stylet 156 remains centered in tool 100 and does not move proximally until a force is applied that exceeds the threshold. The force threshold can be exceeded, for instance, by a surgeon pulling the stylet proximally out of the tool. Accordingly, the plug 702 is a retainer adapted to engage the stylet when the stylet is inserted in the lumen to resist movement of the stylet.

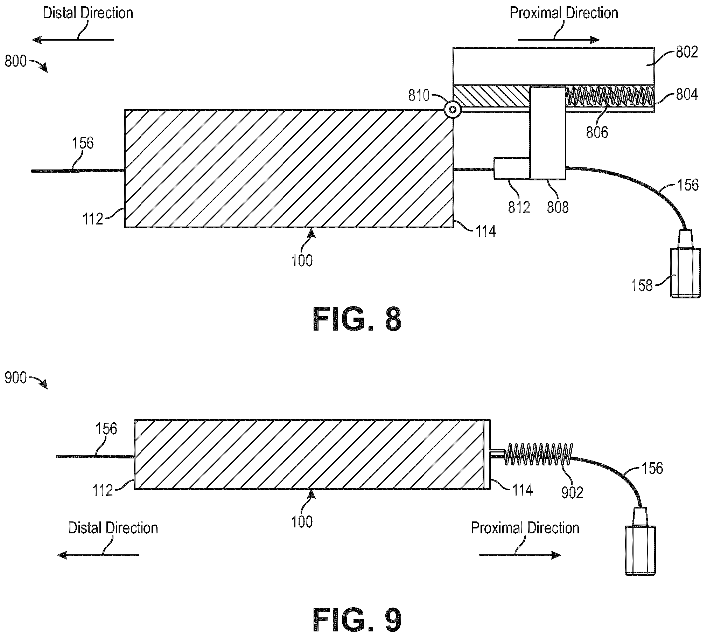

FIG. 8 illustrates an embodiment of a retainer 800 for securing a stylet in a pacing lead tool such as tool 100. Retainer 800 includes a slide body 802 with a slot 804 that runs along a side of the body for some or all of the length of the body. In the illustrated embodiment, the slide body 802 is attached to proximal body end 114 of tool 100 by a hinge 810, so that retainer 800 can be moved from its active position, shown in the drawing, to an inactive position when not needed. But in other embodiments slide body 802 can be attached to tool 100 differently than shown.

A slider 808 is engaged in slot 804 so that the slider can move longitudinally (i.e., proximally and distally) in the slot. In the illustrated embodiment a spring or other elastic member 806 is also positioned in slot 804. Spring 806 is attached to both slide body 802 and slider 808 to provide a biasing force on the slider. In the illustrated embodiment, spring 806 provides a distal biasing force (i.e., a force in the distal direction), but by changing the spring, its position in the slot, or both, the direction of the biasing force can be changed. Slider 808 includes a grip 812 that engages stylet 156, so that the biasing force applied to slider 808 by spring 806 is transmitted to the stylet through the slider and the grip. In another embodiment, slider 808 can retain the stylet by engaging cap 158, and still other embodiments can retain the stylet by using grip 812 to engage the stylet and also using slider 808 to engage cap 158.

In operation of retainer 800, it can either be attached to proximal body end 114 or, if already attached to tool 100 by hinge 810, can be rotated into active position as shown in the drawing. Stylet 156 is then attached to grip 812. As with the inserts and other retainers described above, retainer 800 allows the stylet to move distally while reducing or eliminating proximal movement of the stylet until a proximal force applied to the stylet-e.g., by the weight of the part of the stylet that emerges from proximal body end 114 and, if present, stylet cap 158, and PSA cable 162-exceeds a certain threshold. Thus, stylet 156 remains centered in tool 100 and does not move proximally until a force is applied that exceeds the threshold. The force threshold can be exceeded, for instance, by a surgeon pulling the stylet proximally out of the tool. Accordingly, the grip 812 is a retainer adapted to engage the stylet when the stylet is inserted in a lumen of the tool 100 to resist movement of the stylet.

FIG. 9 illustrates an embodiment of a retainer 900 for securing a stylet in a pacing lead tool. Retainer 900 is attached to the proximal body end 114 of a pacing lead tool such as tool 100. Retainer 900 can be attached to proximal body end 114 differently in different embodiments. In one embodiment, retainer 900 can be attached with adhesives or fasteners. But in other embodiments retainer 900 can be incorporated into tool 100; for instance, in an embodiment where tool 100 is made of molded plastic, retainer 900 can be directly incorporated into the tool by overmolding it into a proximal portion of the tool.

Retainer 900 includes a snare 902 secured to proximal body end 114. In the illustrated snare 902 is a coil; in one embodiment the coil can be formed of metal wire, but in other embodiments it can be formed differently, for example of a non-metal. In operation stylet 156 can be inserted in coil 902 so that the coil engages stylet 156. As with the inserts and other retainers described above, snare 902 allows the stylet to move distally while reducing or eliminating proximal movement of the stylet until a proximal force applied to the stylet-e.g., by the weight of the part of the stylet that emerges from proximal body end 114 and, if present, stylet cap 158, and PSA cable 162-exceeds a certain threshold. Thus, stylet 156 remains centered in tool 100 and does not move proximally until a force is applied that exceeds the threshold. The force threshold can be exceeded, for instance, by a surgeon pulling the stylet proximally out of the tool. Accordingly, the snare 902 is a retainer adapted to engage the stylet when the stylet is inserted in a lumen of the tool 100 to resist movement of the stylet.

An aspect relates to a pacing lead tool 100, 200 comprising a tool body 102 having a proximal body end 114, a distal body end 112, and a tool lumen 110 extending from the proximal body end 114 to the distal body end 112. The tool lumen 110 is adapted to receive a proximal lead end of a pacing lead 152 and a stylet 156 therein. The pacing lead tool 100, 200 also comprises a retainer 202, 300, 310, 400, 450, 500, 600, 700, 800, 900 coupled to the tool body 102 or formed in the tool body 110 and adapted to engage the stylet 156 when the stylet 156 is inserted in the tool lumen 110 to resist movement of the stylet 156.

In an embodiment, the retainer 202, 300, 310, 400, 450, 500, 800, 900 is coupled to the tool body 102 and is adapted to engage the stylet 156 when the stylet 156 is inserted in the tool lumen 110 to resist movement of the stylet 156.

In another embodiment, the retainer 600, 700 is formed in the tool body 110 and is adapted to engage the stylet 156 when the stylet 156 is inserted in the tool lumen 110 to resist movement of the stylet 156.

The above description of embodiments is not intended to be exhaustive or to limit the invention to the described forms. Specific embodiments of, and examples for, the invention are described herein for illustrative purposes, but various modifications are possible.

## Claims

1. A pacing lead tool (100, 200), comprising:
a tool body (102) having a proximal body end (114), a distal body end (112), and a tool lumen (110) extending in a longitudinal direction from the proximal body end (114) to the distal body end (112), wherein the tool lumen (110) is adapted to receive a proximal lead end of a pacing lead (152) and a stylet (156) therein; and
a retainer (202, 300, 310, 400, 450, 500, 600, 700, 800, 900) coupled to the tool body (102) and adapted to engage the stylet (156) when the stylet (156) is inserted in the tool lumen (110) to resist movement of the stylet (156).

2. The pacing lead tool of claim 1, wherein the retainer (202, 300, 310, 400, 450, 500, 600, 700, 800, 900) is adapted to prevent movement of the stylet (156) until a force applied in the longitudinal direction to the stylet (156) exceeds a threshold force.

3. The pacing lead tool of claim 1 or 2, wherein the retainer (202, 300, 310, 400, 450, 500, 600, 700, 800, 900) is an insert (202, 300, 400, 450) positioned in the tool lumen (110).

4. The pacing lead tool of claim 3, wherein the insert (202, 300, 400, 450) is positioned in a notch (206) formed in the tool lumen (110).

5. The pacing lead tool of claim 3 or 4, wherein the insert (300) comprises:
a cannular body (302); and
a membrane (310) within the cannular body (302), the membrane (310) including an opening (312) adapted to receive the stylet (156).

6. The pacing lead tool of claim 5, wherein the membrane (310) has a distal slant.

7. The pacing lead tool of claim 5 or 6, wherein the cannular body (302) and the membrane (310) include a notch (314) to allow the stylet (156) to be removed from the insert (300) and from the pacing lead tool (100, 200).

8. The pacing lead tool of claim 1 or 2, wherein the retainer (500, 600, 700, 800, 900) is attached to the proximal body end (114) of the tool body (102).

9. The pacing lead tool of claim 8, wherein the retainer (900) comprises a snare (902).

10. The pacing lead tool of claim 8, wherein the retainer (500) comprises a pair of arms (502), each arm (502) of the pairs of arms (502) being rotatable about a hinge (504) between an open position and a closed position, and wherein the pair of arms (502) is adapted to engage the stylet (156) when both arms (502) of the pair of arms (502) are in the closed position.

11. The pacing lead tool of claim 8, wherein the retainer (800) comprises:
a slide body (802) attached to the pacing lead tool (100, 200), the slide body (802) including a slot (804);
a slider (808) slidably engaged in the slot (804), wherein the slider (808) is adapted to move in the longitudinal direction in the slot (804), and wherein the slider (808) is adapted to engage a cap (158) attached to a proximal stylet end of the stylet (156) or wherein the slider (808) includes a grip (812) to engage the stylet (156); and
an elastic member (806) positioned in the slot (804) and attached to the slide body (802) and the slider (808), wherein the elastic member (806) is adapted to restrict motion of the cap (158) via the slider (808) or restricts motion of the stylet (156) via the slider (808) and the grip (812).

12. The pacing lead tool of claim 1 or 2, wherein the retainer (600, 700) is formed in the pacing lead tool (100, 200).

13. A pacing lead system (150), comprising:
a pacing lead (152) having a proximal lead end, a distal lead end, and a lead lumen extending from the proximal lead end to the distal lead end; and
a pacing lead tool (100, 200) of any one of claims 1 to 12, wherein the proximal lead end is received in the tool lumen (110).

14. The pacing lead system of claim 13, further comprising a stylet (156) positioned in the lead lumen of the pacing lead (152) and the tool lumen (110) of the tool body (102).

15. The pacing lead system of claim 14, wherein the stylet (156) has a distal stylet end that is positioned at or near the distal lead end of the pacing lead (152) and has a proximal stylet end that is positioned outside the proximal tool end (114) of the tool body (110), and wherein the retainer (202, 300, 310, 400, 450, 500, 600, 700, 800, 900) is configured to engage the stylet (156) to resist movement of the stylet (156).
